# EUROPEAN PATENT SPECIFICATION

(11) **EP 1 686 972 B1**
(45) Date of publication and mention of the grant of the patent: **12.03.2008**
(21) Application number: 04803732.9
(22) Date of filing: 17.11.2004
(51) Int. Cl.: A61K 31/09, A61K 47/14, A61K 47/24, A61K 47/44, A61P 17/06

(54) **SPRAYABLE COMPOSITION FOR THE ADMINISTRATION OF VITAMIN D DERIVATIVES**
SPRÜHBARE ZUSAMMENSETZUNG ZUR VERABREICHUNG VON VITAMIN D DERIVATEN
COMPOSITION PULVERISABLE DESTINEE A ADMINISTRER DES DERIVES DE LA VITAMINE D

(30) Priority: 21.11.2003 FR 0313660; 04.02.2004 US 541245 P
(43) Date of publication of application: 09.08.2006
(60) Divisional application: 08100323.8
(73) Proprietor: GALDERMA RESEARCH & DEVELOPMENT, 06410 Biot (FR)
(72) Inventor: PITRE, Franck, F-77600 Bussy Saint Georges (FR); FREDON, Laurent, Chemin du Camouyer-Lotissement, F-06330 Roquefort les Pins (FR); MALLARD, Claire, F-06250 Mougins (FR)
(74) Representative: Allab, Myriam
(86) International application number: PCT/EP2004/014085
(87) International publication number: WO 2005/053666

(56) References cited:
- EP-A- 0 512 814
- EP-A- 0 966 972
- WO-A-00/26167
- WO-A-97/15295
- WO-A-20/04039390
- WO-A-20/04069134
- FR-A- 2 785 284
- US-A- 4 678 663
- US-A- 4 889 845
- US-B2- 6 538 039
- PATENT ABSTRACTS OF JAPAN vol. 0143, no. 90 (C-0751), 23 August 1990 (1990-08-23) & JP 02 145512 A (SHIN ETSU CHEM CO LTD), 5 June 1990 (1990-06-05)

## Description

The invention relates to a composition comprising a pharmaceutical active agent, at least one volatile silicone and a nonvolatile oily phase in a physiologically acceptable medium, to the process for preparing it and to its use in cosmetics and in dermatology, the composition making it possible to obtain good penetration of the active agent through the layers of the skin.

In the field of dermatology and of the formulation of pharmaceutical compositions, those skilled in the art are led to search for compositions which make it possible to release the active agent and to promote its penetration through the layers of the skin in order to improve its effectiveness. The product should also show good cosmeticity and preferably be nonirritant..

There are currently many topical compositions comprising an active agent and making it possible to promote penetration thereof into the skin by means of the presence in particular of a high content of pro-penetrating glycol. These compositions are formulated in the form of emulsions with a high content of fatty phase, which are commonly called "lipocreams", in the form of anhydrous compositions which are called "ointments", in the form of fluid compositions with a high content of volatile solvents, such as ethanol or isopropanol, intended for application to the scalp, also called "hair lotions", or else in the form of viscous O/W emulsions, which are also called "O/W creams".

O/W creams comprising a corticoid and a high percentage of propylene glycol (47.5%), sold under the trade mark TEMOVATE® by the company GLAXOSMITHKLINE, are, for example, known. The stabilizing of a formulation comprising such a percentage of glycol makes it necessary to use, in the emulsion, emulsifiers and stabilizers of the glyceryl stearate or PEG 100 stearate type, or alternatively stabilizers or consistency factors of the white wax or cetostearyl alcohol type, which result in the formation of a viscous cream, that is to say a cream with a viscosity greater than 10 Pa.s (10 000 centipoises, measured with a Brookfield model LVDV II + mobile No. 4 device, at a rate of 30 rpm for 30 seconds and at a temperature of 25°C ± 3°C). This viscosity therefore makes the product difficult to apply. These compositions therefore show, firstly, poor cosmetic acceptability due to their viscosity and, secondly, risks of intolerance caused by the presence of high proportions of glycol. Those skilled in the art therefore wish to improve these parameters, by virtue of the present invention.

In order to facilitate the application of topical compositions comprising a high percentage of pro-penetrating glycol, the applicant has produced, and protected by means of application EP 832647, a lotion, which is a stable formulation of O/W emulsion type, and the viscosity of which is intermediate between hair lotions which are too fluid and have too limited a use, and O/W creams which are too viscous and have a greasy and sticky side to them, while at the same time conserving the pro-penetrating properties of the glycol. These formulae effectively show good penetration of the active agent, but still comprise a high percentage of glycol which can therefore induce a sticky effect or problems of tolerance resulting in moderate acceptability of the product by the patient.

Formulations containing silicone compounds which result in compositions which are pleasant to use are, moreover, known to those skilled in the art. Thus, in US patent 6,538,039, a novel formulation of active agent for transdermal administration has been developed, comprising silicone compounds in order to deposit a film at the surface of the skin. In that application also, the transdermal passage is facilitated by the obligatory presence of absorption promoters, namely, among other compounds mentioned, glycols.

In patent application EP 0966972, the compositions described can be formulated in the form of a spray and comprise an active compound, a silicone gum and a pharmaceutically acceptable excipient. The problem that the invention described in EP 0966972 proposes to solve i s that of depositing a substantive film at the surface of the skin, which problem is solved by means of the presence of the silicone qum.

The problem that the present invention here proposes to solve is that of designing a composition tor improving the penetration of the pharmaceutical active agent, and its rapidity of penetration over time, in order Lo improve its therapeutic efficacy, while at the same time avoiding the presence of a high content of glycol. The composition according to the invention should also be easy to use and show a cosmeticity which is acceptable for application to all the regions of the body which may be affected by the pathology.

The two applications EP 0966972 and US 6, 538, 039 represent Lhe prior art closest to the present invention, given the composition of the formulations described. However, on reading this prior art, there is nothing which could prompt those skilled in the art to choose the composition according to the invention in order to obtain good penetration of the active agent incorporated, into the layers of the skin. WO97/15295 describes sprayable compositions for transdermal delivery comprising a lipophilic active agent.

EP0512814 describes compositions comprising a derivative of cholecalciferol and a sunscreen material. US4, 889, 845 describes a vehicle for the topical application of prostaglandins, and US4,678,663 describes hydroquinone compositions.

JPO2145512 describes compositions comprising an active principle, a cyclic volatile silicone and a film-forming agent. Finally, FR2785284 and WO00/26167 describe analogs of vitamin D.

In fact, the applicant has found, surprisingly, that the composition comprising, in a pharmaceutically acceptable vehicle:
a) a therapeutically effective amount of a pharmaceutical active agent,
b) at least one volatile silicone,
c) a nonvolatile oily phase,
results in an improvement in penetration of the active agents described below.

The composition of the present invention, while allowing good penetration of the active principles, also shows very good acceptability and tolerance among patients, as described in Examples 8 and 9 of the present invention. It is therefore found that the composition according to the invention is particularly suitable for the treatment of dermatological conditions, and more particularly very suitable for the treatment of psoriasis.

The invention relates more particularly to a composition comprising, in a pharmaceutically acceptable vehicle:
a) a therapeutically effective amount of a pharmaceutical active agent,
b) at least one volatile silicone,
c) a nonvolatile oily phase,
characterized in that the pharmaceutical active agent is a compound derived from vitamin D as described below.

The term "compound derived from vitamin D" is intended to mean the following:
1. 6-[3-(3,4-bishydroxymethylbenzyloxy)phenyl]-2-methylhepta-3,5-dien-2-ol,
2. 7-[3-(3,4-bishydroxymethylphenoxymethyl)phenyl]-3-ethyloctan-3-ol,
3. 7-{3-[2-(3,4-bishydroxymethylphenyl)ethyl]phenyl}-3-ethylocta-4,6-dien-3-ol,
4. 6-{3-[2-(3,9-bishydroxymethylphenyl)ethyl]phenyl}-2-methylhepta-3,5-dien-2-ol,
5. 7-{3-[2-(3,4-bishydroxymethylphenyl)vinyl]phenyl}-3-ethylocta-4,6-dien-3-ol,
6. 7-[3-(3,4-bishydroxymethylbenzyloxy)phenyl]-3-ethyl-3-octanol,
7. 4E, 6E)-7-[3-(3,4-bishydroxymethylbenzyloxy)-phenyl]-3-ethylocta-4,6-dien-3-ol,
8. (4E,6E)-7-[3-(3,4-bishydroxymethylbenzyloxy)-phenyl]-3-ethylnona-4,6-dien-3-ol,
9. (E)-7-[3-(3,4-bishydroxymethylbenzyloxy)phenyl]-3-ethyloct-4-en-3-ol,
10. (E)-7-[3-(3,4-bishydroxymethylbenzyloxy)phenyl]-3-ethyloct-6-en-3-ol,
11. (E)-7-[3-(3,4-bishydroxymethylbenzyloxy)phenyl]-3-ethyloct-6-en-4-yn-3-ol,
12. (4E,6E)-7-[3-(3,4-bishydroxymethylphenoxymethyl)-phenyl]-3-ethylocta-4,6-dien-3-ol,
13. (E)-7-[3-(3,4-bishydroxymethylphenoxymethyl)-phenyl]-3-ethylnon-6-en-3-ol,
14. (E)-7-{3-[(3,4-bishydroxymethylbenzyl)methylamino]phenyl}-3-ethyloct-6-en-3-ol,
15. 7-[3-(3,4-bishydroxymethylbenzyloxy)phenyl]-3-ethyl-7-methyloctan-3-ol,
more preferably, the pharmaceutical active agent incorporated into the composition according to the invention is (4E, 6E)-7-[3-(3,4-bishydroxymethyl-benzyloxy)phenyl]-3-ethylnona-4,6-dien-3-ol.

Advantageously, the composition according to the invention comprises between 0.0001 and 20% by weight, relative to the total weight of the composition, of an active agent, preferably between 0.025 and 15% by weight, and more preferably between 0.01 and 5% by weight.

Of course, the amount of active agent in the composition according to the invention will depend on the active agent under consideration.

The composition according to the invention will preferably comprise an active agent derived from vitamin D at a concentration of less than 2% by weight of active agent, preferably of between 0.025 and 0.5% by weight. The preferred pharmaceutical active agent according to the invention is (4E, 6E)-7-[3-(3,4-bishydroxymethylbenzyloxy)phenyl]-3-ethylnona-4,6-dien-3-ol used at a concentration of 0.3% by weight.

The active agents which can be used according to the invention may be used alone or in combination.

According to the invention, the term "volatile silicone" is intended to mean polyorganosiloxane compounds, which may be cyclic or linear, having a measurable pressure under ambient conditions. The cyclic volatile silicones according to the invention are polydimethylcyclosiloxanes, i.e. compounds of formula: 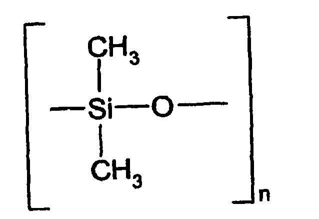 with n being, on average, between 3 and 6, and preferably n=4 or n=5, generally known as cyclomethicones. The linear volatile silicones according to the invention are linear polysiloxanes such as hexamethyldisiloxane or low molecular weight dimethicones. The linear volatile silicones generally have a viscosity of less than approximately 5 centistokes at 25°Celsius, whereas the cyclic volatile silicones have a viscosity of less than approximately 10 centistokes at 25° Celsius.

Preferred volatile silicones according to the invention are the linear siloxanes, and more preferably hexamethyldisiloxane. By way of example, mention may be made of the product sold by the company DOW CORNING, DC Fluid 0.65cSt.

Advantageously, the composition according to the invention comprises between 25 and 95% by weight, relative to the total weight of the composition, of the volatile silicone, and preferably between 40 and 80% by weight, and more preferably between 55 and 65% by weight.

According to the invention, the term "nonvolatile oily phase" is intended to mean a variety of nonvolatile oil suitable for a pharmaceutical or cosmetic composition. The nonvolatile oils generally have a viscosity of greater than approximately 10 centipoises at 25°C, and can reach a viscosity ranging up to 1 000 000 centipoises at 25°C. The nonvolatile oily phase can be made up of a large variety of synthetic or natural, silicone or organic oils, a nonexhaustive list of which is given by way of indication.

### (a) Esters

Examples of a nonvolatile oil which can be used according to the invention comprise esters of formula RCO-OR' with R and R', which may be identical or different, representing a linear or branched chain of an alkyl, alkenyl, alkoxycarbonylalkyl or alkoxycarbonyloxyalkyl containing from 1 to 25 carbon atoms, preferably from 4 to 20 carbon atoms. Examples of such esters include isotridecyl isononanoate, PEG-4 diheptanoate, isostearyl neopentanoate, tridecyl neopentanoate, cetyl octanoate, cetyl palmitate, cetyl ricinoleate, cetyl stearate, cetyl myristate, coco dicaprylate/caprate, decyl isostearate, isodecyl oleate, isodecyl neopentanoate, isohexyl neopentanoate, octyl palmitate, dioctyl malate, tridecyl octanoate, myristyl myristate and octododecanol.

### (b) Glyceryl esters of fatty acids

The oil may also comprise fatty esters of natural fatty acids, or triglycerides of animal or plant origin. Such examples include, castor oil, lanolin oil, triisocetyl citrate, triglycerides containing from 10 to 18 carbon atoms, caprylic/capric triglycerides, coconut oil, corn oil, cottonseed oil, flax oil, mink oil, olive oil, palm oil, illipe butter, rapeseed oil, soybean oil, sunflower oil, nut oil and equivalent.

### (c) Fatty acid glycerides

The oils which are also suitable are synthetic or semi-synthetic glyceryl esters, such as fatty acid mono-, di or triglycerides, which are modified natural oils or fats, for example glyceryl stearate, glyceryl dioleate, glyceryl distearate, glyceryl trioctanoate, glyceryl linoleate, glyceryl myristate, glyceryl isostearate, PEG castor oils, PEG glyceryl oleates, PEG glyceryl stearates, and equivalent.

### (d) Nonvolatile hydrocarbons

Nonvolatile hydrocarbons such as paraffins, isoparaffins, mineral oils, and equivalent are also very suitable for the composition according to the invention, as non-volatile nonpolar solvent.

### (e) Guerbet esters

Guerbet esters are esters resulting from the reaction of a Guerbet alcohol of general formula: 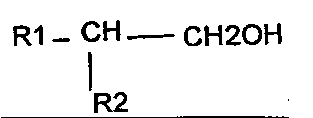 and a carboxylic acid of general formula R3-COOH or HOOC-R3-COOH,
R1 and R2, which may be identical or different, represent an alkyl containing from 4 to 20 carbon atoms, and R3 represents a substituted or unsubstituted fatty radical, such as a linear or branched, saturated or unsaturated alkyl or alkylene chain containing from 1 to 50 carbon atoms, a phenyl, which may be substituted with a halogen, a hydroxyl, a carboxyl, or an alkylcarbonylhydroxyl.

### (f) Silicone oils

The silicone oils which can be used according to the invention for making up the nonvolatile phase are polyorganosiloxane compounds having a measurable pressure under ambient conditions and a viscosity strictly greater than 10 centistokes and lower than 20 centistokes. The nonvolatile silicones which can be used according to the invention are the compounds of formula: 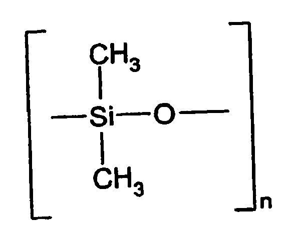 with n strictly greater than 6.

The preferred nonvolatile oily phase according to the invention is paraffin oil.

Advantageously, the composition according to the invention comprises between 1 and 50% by weight, relative to the total weight of the composition, of nonvolatile oily phase, preferably between 5 and 30% by weight, and more preferably between 7 and 15% by weight.

According to a preferred embodiment of a composition according to the invention, the composition also comprises a silicone gum. The applicant has, in fact, discovered, surprisingly, that a composition comprising a silicone gum in the concentrations defined hereinafter shows more rapid penetration of the active agent through the various layers of the skin.

The term "silicone gums" is intended to mean the silicone gums known to those skilled in the art, and in particular those described in patent application EP 0966972, incorporated herein by way of reference. According to this preferred embodiment of a composition according to the invention, the silicone gum is introduced at a concentration of between 0.001 and 3% by weight, preferably between 0.01 and 1% by weight. Dow Corning provides a commercial product sold under the name DC Silmogen Carrier, which is made up of 99% of hexamethyldisiloxane and 1% of silicone gum, which product may advantageously be used in one of the compositions according to the invention.

The pharmaceutically acceptable vehicle according to the invention should be chosen such that the advantageous properties intrinsically associated with the present invention are not, or are not substantially, altered by the envisaged addition. Preferably, the vehicle used according to the invention is chosen so as to be an agent which solubilizes the active agent. The active agent-solubilizing vehicle may be made up of a single excipient, such as a solvent, or of a mixture of excipients, such as those used for the formulation of an emulsion. By way of nonlimiting examples of excipients which may be used alone or as a mixture, mention may be made of water, solvents, diluents, and any excipient which can be used for the formulation of an emulsion, of a milk, of a gel, of an ointment, or of a foaming composition. These excipients are compounds commonly used in the formulation of a pharmaceutical composition. Preferably, the active agent-solubilizing excipients according to the invention are water, alcohols, polyols, ethers, esters, aldehydes, ketones, fatty acids and fatty alcohols, and fatty esters. More preferably, the excipient used will be an alcohol. According to the invention, the term "alcohol" is intended to mean linear or branched aliphatic alcohols such as ethanol, propanol or isopropanol.

In a preferred embodiment according to the invention, the vehicle used will therefore be alcoholic.

According to the invention, the term "alcoholic vehicle" is intended to mean a vehicle comprising at least 15% of alcohol, and preferably at least 25% of ethanol.

The pharmaceutical composition according to the invention may also contain inert additives or combinations of these additives, such as:
- wetting agents;
- flavour enhancers;
- preserving agents;
- stabilizers;
- moisture regulators;
- pH regulators;
- osmotic pressure modifiers;
- emulsifiers;
- UV-A and UV-B screening agents;
- propenetrating agents;
- antioxidants;
- and synthetic polymers.

Of course, those skilled in the art will take care to choose the possible compound(s) to be added to these compositions in such a way that the advantageous properties intrinsically associated with the present invention are not, or are not substantially, altered by the envisaged addition.

The composition according to the invention is more particularly intended for treating the skin and the mucous membranes, and may be provided in the form of ointments, creams, milks, salves, powders, impregnated pads, syndets, solutions, gels, sprays, foams, suspensions, lotions, sticks, shampoos, pledgets or washing bases. It may also be provided in the form of suspensions of lipid or polymer vesicles or nanospheres or microspheres or polymer patches and hydrogels to allow controlled release. This topical-application composition may be provided in anhydrous form, in aqueous form or in the form of an emulsion.

The composition according to the invention showing improved penetration is preferably administered in the form of a sprayable composition. In order to be sprayable, the compositions according to the invention will preferably have a viscosity of less than 50 centistokes, and more preferably less than 10 centistokes.

The sprayable form, or spray, can be obtained by conventional formulation means known to those skilled in the art. For example, the composition may be sprayed by means of a mechanical spraying device which pumps the composition from a container, bottle or equivalent. The composition passes through a nozzle which can be aimed directly at the desired site of application. The nozzle can be chosen so as to apply the composition in the form of a vaporization or of a jet of droplets, according to techniques known to those skilled in the art. According to the pharmaceutical active agent chosen, the spraying mechanism must be capable of always delivering the same amount of active agent. The mechanisms for controlling the amount of composition to be delivered by the spray are also known to those skilled in the art.

Preferably, for the composition according to the invention, a dosing spray bottle, for which the application area and dose characteristics are controlled and reproducible, will be used. For example, the spray device used consists of a bottle equipped with a 25 µl dosing valve.

A subject of the present invention is also the use of a composition according to the invention, for producing a medicinal product intended for treating:
- dermatological conditions associated with a keratinization disorder relating to differentiation and to proliferation, in particular common acne, comedo-type acne, polymorphic acne, rosacea, nodulocystic acne, acne conglobata, senile acne, and secondary acne such as solar, drug-related or occupational acne,
- ichthyoses, ichthyosiform conditions, Darrier's disease, palmoplantar keratoderma, leukoplakia and leukoplakiform conditions, and cutaneous or mucosal (oral) lichen,
- dermatological conditions with an inflammatory immunoallergic component, with or without a cell proliferation disorder, in particular cutaneous, mucosal or ungual psoriasis, psoriatic rheumatism, cutaneous atopy, such as eczema, respiratory atopy or gingival hypertrophy,
- benign or malignant dermal or epidermal proliferations, of viral or nonviral origin, in particular common warts, flat warts, epidermodysplasia verruciformis, oral or florid papillomatoses, and T lymphoma,
- proliferations which may be induced by ultraviolet light, in particular basal cell epithelioma and spinocellular epithelioma,
- precancerous skin lesions, in particular keratoacanthomas,
- immune dermatoses, in particular lupus erythematous,
- bullous immune diseases,
- collagen diseases, in particular scleroderma,
- dermatological or systemic conditions with an immunological component,
- skin disorders due to exposure to UV radiation, or light-induced or chronological ageing of the skin, or actinic keratoses and pigmentations, or any pathologies associated with chronological or actinic ageing, in particular xerosis,
- sebaceous function disorders, in particular hyperseborrhoea acne or simple seborrhoea or seborrhoeic dermatitis,
- cicatrization disorders or stretch marks,
- pigmentation disorders, such as hyperpigmentation, melasma, hypopigmentation or vitiligo,
- lipid metabolism conditions, such as obesity, hyperlipidemia, non-insulin-dependant diabetes or syndrome X,
- inflammatory conditions such as arthritis,
- cancerous or precancerous states,
- alopecia of various origins, in particular alopecia caused by chemotherapy or radiation,
- immune system disorders, such as asthma, type 1 diabetes mellitus, multiple sclerosis or other selective dysfunctions of the immune system, or
- cardiovascular system conditions such as arteriosclerosis or hypertension.

In a preferred embodiment of use of the composition, said composition will contain 0.3% of (4E,6E)-7-[3-(3,4-bishydroxymethylbenzyloxy)phenyl]-3-ethylnona-4,6-dien-3-ol and will be used for producing a medicinal product intended to treat psoriasis.

The invention also relates to a process for improving the penetration of an active agent derived from vitamin D, characterized in that a composition comprising the following, in a pharmaceutically acceptable vehicle, is applied to the skin:
a) a therapeutically effective amount of an active agent derived from vitamin D,
b) at least one volatile silicone,
c) a nonvolatile oily phase,
said composition being applied in the form of a spray.

In fact, the applicant has discovered, surprisingly, that the penetration of compounds derived from vitamin D, through the skin is improved by the composition according to the invention, even in the absence of excipients with propenetrating activity.

The expression "improvement in penetration into the skin" is intended to mean a significant increase in penetration into the skin of at least a factor of 2, compared to formulations previously produced on the market.

The penetration of the active agent is measured according to the protocol described in Example 4.

The following examples show, in a nonexhaustive manner, examples of formulation of the composition according to the invention and results of penetration into the skin.

### Example 1:

The formulation is obtained by mixing the various compounds mentioned below until a homogeneous and clear solution is obtained.

| Ingredients | Function | Spray A |
|---|---|---|
| (4E,6E)-7-[3-(3,4-bis-hydroxymethylbenzyloxy)phenyl]-3-ethylnona-4,6-dien-3-ol | Active agent | 0.3% |
| Hexamethyldisiloxane | Volatile silicone | 60.0% |
| Paraffin oil | Nonvolatile oily phase | 10.0% |
| Absolute ethanol | Solvent: excipient | qs 100% |

### Example 2:

The procedure used is the same as that in Example 1.

| Ingredients | Function | Spray B |
|---|---|---|
| (4E,6E)-7-[3-(3,4-bis-hydroxymethylbenzyloxy)phenyl]-3-ethylnona-4,6-dien-3-ol | Active agent | 0.3% |
| Hexamethyldisiloxane | Volatile silicone | 59.4% |
| Silicone gum | Silicone gum | 0.6% |
| Paraffin oil | Nonvolatile oily phase | 10.0% |
| Absolute ethanol | Solvent | qs 100% |

### Example 3:

The procedure used is the same as that in Example 1.

| Ingredients | Function | Spray C |
|---|---|---|
| (4E,6E)-7-[3-(3,4-bis-hydroxymethylbenzyloxy)phenyl]-3-ethylnona-4,6-dien-3-ol | Active agent | 0.3% |
| Hexamethyldisiloxane | Volatile silicone | 59.4% |
| Silicone gum | Silicone gum | 0.6% |
| Paraffin oil | Nonvolatile oily phase | 10.0% |
| Oleic acid | Propenetrating agent | 5.0% |
| Butylhydroxytoluene (BHT) | Antioxidant | 0.05% |
| Absolute ethanol | Solvent | qs 100% |

Example 4: Study of the release/penetration *in vitro,* on human skin, of (4E,6E)-7-[3-(3,4-bishydroxymethylbenzyloxy)phenyl]-3-ethylnona-4,6-dien-3-ol contained in two different formulations, one of which is sprayable according to the preferred embodiment of the invention.

The first aim is to quantify the penetration into the skin of the active agent formulated in both formulations, *in vitro*, on human skin, after 16 hours of application. A sprayable formula according to the invention is compared with a composition in the form of an ointment.

The exact compositions of the two formulations are given in Table 1 below.

**TABLE 1**

| Ingredients | Function | Spray (in %) | Ointment (in %) |
|---|---|---|---|
| (4E,6E)-7-[3-(3,4-bishydroxymethylbenzyloxy)-phenyl]-3-ethylnona-4,6-dien-3-ol | Active agent | 0.3 | 0.3 |
| Hexamethyldisiloxane | Volatile silicone | 59.40 | |
| Silicone gum | Silicone gum | 0.6 | |
| Paraffin oil | Occlusive agent | 10.00 | 5 |
| Oleic acid | Propentrating agent | / | / |
| Propylene glycol | Propentrating agent | | 10.00 |
| White petroleum jelly | Occlusive agent | | 76.94 |
| Macrogol 2 stearyl ether | | | 5 |
| Disodium EDTA | Chelating agent | | 0.0065 |
| DL-alpha-tocopherol | Antioxidant | | 0.12 |
| BHT | Antioxidant | | |
| Absolute ethanol | Solvent | qs 100% | |
| Water | Solvent | | qs 100% |

**Experimental conditions:** Percutaneous absorption is evaluated by means of diffusion cells consisting of 2 compartments separated by human skin. The formulations were applied without occlusion for 16 hours. The formulations were applied at a rate of 10 mg of formulation per cm² (i.e. 30 micrograms of (4E,6E)-7-[3-(3,4-bishydroxymethylbenzyloxy)phenyl]-3-ethylnona-4,6-dien-3-ol). Throughout the duration of the study, the dermis is in contact with a recipient liquid which is not renewed as a function of time (static mode). At the end of the application period, the surface excess is removed and the distribution of the (4E,6E)-7-[3-(3,4-bishydroxymethylbenzyloxy)phenyl]-3-ethylnona-4,6-dien-3-ol is quantified in the various skin compartments and in the recipient liquid. The concentrations of (4E,6E)-7-[3-(3,4-bishydroxymethylbenzyloxy)phenyl]-3-ethylnona-4,6-dien-3-ol were quantified using an HPLC/MS/MS method conventionally known to those skilled in the art (LQ: 10 ng.mL⁻¹). The spray formula was applied using a spray bottle equipped with a 25 µl dosing valve.

The experimental results show that, whichever the formulation tested, the active agent is distributed mainly in the skin (epidermis, including strateum corneum, and dermis). The total amounts penetrated (stratum corneum + epidermis + dermis + recipient liquid) are:

| | Application time: 16 hours |
|---|---|
| Spray | |
| Total amount having penetrated | |
| - µg | 2.64 ± 0.50 µg |
| - % dose applied | 9.2% |
| Ointment | |
| Total amount having penetrated | |
| - µg | 0.63 ± 0.14 µg |
| - % dose applied | 2.3% |

### Results:

The results show here that the spray formula according to the invention shows a four-fold increase in penetration of the active agent after 16 hours, compared with the ointment formula.

This result therefore indicates that the compositions according to the invention make it possible to obtain a significant improvement in penetration of an active agent derived from vitamin D compared with existing formulas.

The spray formulas as described therefore make it possible to do without the use of glycols, without decreasing skin penetration, and therefore show an additional advantage in terms of nonirritant potential versus the compositions comprising a high content of glycol.

## Claims

1. composition comprising, in a pharmaceutically acceptable vehicle:
a) a therapeutically effective amount of at least one pharmaceutical active agent,
b) at least one volatile silicone,
c) a nonvolatile oily phase,
**characterized in that** the composition is sprayable, and **in that** the pharmaceutical active agent is derived from vitamin D and is chosen from the group consisting of:
6-[3-(3,4-bishydroxymethylbenzyloxy)phenyl]-2-methylhepta-3,5-dien-2-ol,
7-[3-(3,4-bishydroxymethylphenoxymethyl)phenyl]-3-ethyloctan-3-ol,
7-{3-[2-(3,4-bishydroxymethylphenyl)ethyl]phenyl}-3-ethylocta-4,6-dien-3-ol,
6-{3-[2-(3,4-bishydroxymethylphenyl)ethyl]phenyl}-2-methylhepta-3,5-dien-2-ol,
7-{3-[2-(3,4-bishydroxymethylphenyl)vinyl]phenyl}-3-ethylocta-4,6-dien-3-ol,
7-[3-(3,4-bishydroxymethylbenzyloxy)phenyl]-3-ethyl-3-octanol,
4E, 6E)-7-[3-(3,4-bishydroxymethylbenzyloxy)phenyl]-3-ethylocta-4,6-dien-3-ol,
(4E, 6E)-7-[3-(3,4-bishydroxymethylbenzyloxy)phenyl]-3-ethylnona-4,6-dien-3-ol,
(E)-7-[3-(3,4-bishydroxymethylbenzyloxy)phenyl]-3-ethyloct-4-en·3-ol,
(E) ·7-[3-(3,4-bishydroxymethylbenzyloxy)phenyl]-3-ethyloct-6-en-3-ol,
(E)-7-[3-(3,4-bishydroxymethylbenzyloxy)phenyl]-3-ethyloct-6-en-4-yn-3-ol,
(4E,6E)-7-[3-(3,4-bishydroxymethylphenoxymethyl)phenyl]-3-ethylocta-4,6-dien-3-ol,
(E)-7-[3-(3,4-bishydroxymethylphenoxymethyl)phenyl]-3-ethylnon-6-en-3-ol,
(E)-7-{3-[(3,4-bishydroxymethylbenzyl)methylamino]-phenyl}-3-ethyloct-6-en-3-ol,
7-[3-(3,4-bishydroxymethylbenzyloxy)phenyl]-3-ethyl-7-methyloctan-3-ol.

2. Composition according to Claim 1,
**characterized in that** it comprises:
a) between 0.0001 and 20% by weight of the active agent,
b) between 25 and 95% by weight of volatile silicone,
c) between 1 and 50% by weight of nonvolatile oily phase.

3. Composition according to one of Claims 1 or 2, **characterized in that** the pharmaceutical active agent is (4E, 6E)-7-[3-(3,4-bishydroxymethylbenzyloxy)phenyl]-3-ethylnona-4,6-dien-3-ol.

4. Composition according to one of Claims 1 to 3, **characterized in that** it contains between 0.01% and 2 % by weight of compound derived from vitamin D.

5. Composition according to one of Claims 1 to 4, **characterized in that** the vehicle is an alcoholic vehicle.

6. Composition according to one of Claims 1 to 5, **characterized in that** the alcoholic vehicle comprises at least 15% of alcohol.

7. Composition according to one of Claims 1 to 6, **characterized in that** the alcoholic vehicle comprises at least 25% of ethanol.

8. Composition according to one of Claims 1 to 7, **characterized in that** the volatile silicone is chosen from the group consisting of polydimethylcyclosiloxanes and low molecular weight linear polysiloxanes.

9. Composition according to one of Claims 1 to 8, **characterized in that** the volatile silicone is a linear polysiloxane of the hexamethyldisiloxane type.

10. Composition according to one of Claims 1 to 9, **characterized in that** it contains between 55 and 65% by weight of hexamethyldisiloxane.

11. Composition according to one of Claims 1 to 10, **characterized in that** the nonvolatile oily phase is a nonpolar oil.

12. Composition according to one of Claims 1 to 8, **characterized in that** the nonpolar oil is paraffin oil.

13. Composition according to one of Claims 1 to 9, **characterized in that** it contains between 5 and 15% of paraffin oil.

14. Composition according to any one of the preceding claims, **characterized in that** it also contains a silicone gum.

15. Composition according to any one of Claims 1 to 10, **characterized in that** it contains:
a) 0.3% of (4F,6E)-7-[3-(3,4-bishydroxymethylbenzyloxy)phenyl]-3-ethylnona-4,6-dien-3-ol,
b) 60% of hexamethyldisiloxane,
c) 10% of paraffin oil,
d) 29.7% of ethanol.

16. Composition according to any one of Claims 1 to 11, **characterized in that** it contains:
a) 0.3% of (4E, 6E)-7-[3-(3,4-bishydroxymethylbenzyloxy)phenyl]-3-ethylnona-4,6-dien-3-ol,
b) 59.4% of hexamethyldisiloxane,
c) 0.6% of silicone gum,
d) 10% of paraffin oil,
e) 29.7% of ethanol.

17. Use of a composition according to any one of Claims 1 to 14, for producing a medicinal product intended for treating:
- dermatological conditions associated with a keratinization disorder relating to differentiation and to proliferation, in particular common acne, comedo-type acne, polymorphic acne, rosacea, nodulocystic acne, acne conglobata, senile acne, and secondary acne such as solar, drug-related or occupational acne,
- ichthyoses, ichthyosiform conditions, Darrier's disease, palmoplantar keratoderma, leukoplakia and leukoplakiform conditions, and cutaneous or mucosal (oral) lichen,
- dermatological conditions with an inflammatory immunoallergic component, with or without a cell proliferation disorder, in particular cutaneous, mucosal or ungual psoriasis, psoriatic rheumatism, cutaneous atopy, such as eczema, respiratory atopy or gingival hypertrophy,
- benign or malignant dermal or epidermal proliferations, of viral or nonviral origin, in particular common warts, flat warts, epidermodysplasia verruciformis, oral or florid papillomatoses, and T lymphoma,
- proliferations which may be induced by ultraviolet light, in particular basal cell epithelioma and spinocellular epithelioma,
- precancerous skin lesions, in particular keratoacanthomas,
- immune dermatoses, in particular lupus erythematous,
- bullous immune diseases,
- collagen diseases, in particular scleroderma,
- dermatological or systemic conditions with an immunological component,
- skin disorders due to exposure to UV radiation, or light-induced or chronological ageing of the skin, or actinic keratoses and pigmentations, or any pathologies associated with chronological or actinic ageing, in particular xerosis,
- sebaceous function disorders, in particular hyperseborrhoea acne or simple seborrhoea or seborrhoeic dermatitis,
- cicatrization disorders or stretch marks,
- pigmentation disorders, such as hyperpigmentation, melasma, hypopigmentation or vitiligo,
- lipid metabolism conditions, such as obesity, hyperlipidemia, non-insulin-dependant diabetes or syndrome X,
- inflammatory conditions such as arthritis,
- cancerous or precancerous states,
- alopecia of various origins, in particular alopecia caused by chemotherapy or radiation,
- immune system disorders, such as asthma, type 1 diabetes mellitus, multiple sclerosis or other selective dysfunctions of the immune system, or
- cardiovascular system conditions such as arteriosclerosis or hypertension.

18. Use of a composition, according to Claim 17, for treating psoriasis.

19. Process for improving the penetration of a pharmaceutical active agent derived from vitamin D, **characterized in that** a composition comprising the following, in a pharmaceutically acceptable vehicle is applied to the skin:
a) a therapeutically effective amount of a compound derived from vitamin D,
b) at least one volatile silicone,
c) a nonvolatile oily phase, said composition being applied in the form of a spray.

20. Process according to Claim 19, **characterized in that** the active agent is (4E,6E)-7-[3-(3,4-bishydroxymethylbenzyloxy)phenyl]-3-ethylnona-4,6-dien-3-ol, the volatile silicone is hexamethyldisiloxane, and the hydrophobic solvent is paraffin oil.

21. Process according to either of Claims 19 and 20, **characterized in that** the composition also comprises a silicone gum.

## Patentansprüche

1. Zusammensetzung, die in einem pharmazeutisch akzeptablen Träger enthält:
a) eine pharmazeutisch wirksame Menge mindestens eines pharmazeutischen Wirkstoffes,
b) mindestens ein flüchtiges Silicon,
c) eine nichtflüchtige Ölphase,
**dadurch gekennzeichnet, dass** die Zusammensetzung sprühbar ist, und **dadurch**, dass der pharmazeutische Wirkstoff von Vitamin D abgeleitet und unter den folgenden Verbindungen ausgewählt ist:
6-[3-(3,4-Bis-hydroxymethylbenzyloxy)phenyl]-2-methylhepta-3,5-dien-2,ol,
7-[3-(3,4-Bis-hydroxymethylphenoxymethyl)phenyl]-3-ethyloctan-3-ol,
7-{3-[2-(3,4-Bis-hydroxymethylphenyl)ethyl]phenyl}-3-ethylocta-4,6-dien-3-ol,
6-{3-[2-(3,4-Bis-hydroxymethylphenyl)ethyl]phenyl}-2-methylhepta-3,5-dien-2-ol,
7-{3-[2-(3,4-Bis-hydroxymethylphenyl)vinyl]phenyl}-ethylocta-4,6-dien-3-ol,
7-(3-(3,4-Bis-hydroxymethylbenzyloxy)phenyl]-3-ethyl-3-octanol,
(4E,6E)-7-[3-(3,4-Bis-hydroxymethylbenzyloxy)phenyl]-3-ethylocta-4,6-dien-3-ol,
(4E,6E)-7-[3-(3,4-Bis-hydroxymethylbenzyloxy)phenyl]-3-ethylnona-4,6-dien-3-ol,
(E)-7-[3-[3,4-Bis-hydroxymethylbenzyloxy)phenyl]-3-ethyloct-4-en-3-ol,
(E)-7-[3-(3,4-Bis-hydroxymethylbenzyloxy)phenyl]-3-ethyloct-6-en-3-ol,
(E)-7-[3-(3,4-Bis-hydroxymethylbenzyloxy)phenyl]-3-ethyloct-6-en-4-in-3-ol,
(4E,6E)-7-[3-(3,4-Bis-hydroxymethylphenoxymethyl)phenyl]-3-ethylocta-4,6-dien-3-ol,
(E)-7-[3-(3,4-Bis-hydroxymethylphenoxymethyl)phenyl]-3-ethylnon-6-en-3-ol,
(E)-7-{3-[(3,4-Bis-hydroxymethylbenzyl)methylamino]phenyl)-3-ethyloct-6-en-3-ol,
7-[3-(3,4-Bis-hydroxymethylbenzyloxy)phenyl]-3-ethyl-7-methyloctan-3-ol.

2. Zusammensetzung nach Anspruch 1, **dadurch gekennzeichnet, dass** sie enthält:
a) 0,0001 bis 20 Gew.-% Wirkstoff,
b) 25 bis 95 Gew.-% flüchtiges Silicon,
c) 1 bis 50 Gew.-% nichtflüchtige Ölphase.

3. Zusammensetzung nach einem der Ansprüche 1 oder 2, **dadurch gekennzeichnet, dass** der pharmazeutische Wirkstoff das (4E,6E)-7-[3-(3,4-Bis-hydroxymethylbenzyloxy)phenyl]-3-ethylnona-4,6-dien-3-ol ist.

4. Zusammensetzung nach einem der Ansprüche 1 bis 3, **dadurch gekennzeichnet, dass** sie 0,01 bis 2 Gew.-% der von Vitamin D abgeleiteten Verbindung enthält.

5. Zusammensetzung nach einem der Ansprüche 1 bis 4, **dadurch gekennzeichnet, dass** der Träger ein alkoholischer Träger ist.

6. Zusammensetzung nach einem der Ansprüche 1 bis 5, **dadurch gekennzeichnet, dass** der alkoholische Träger mindestens 15 % Alkohol enthält.

7. Zusammensetzung nach einem der Ansprüche 1 bis 6, **dadurch gekennzeichnet, dass** der alkoholische Träger mindestens 25 % Ethanol enthält.

8. Zusammensetzung nach einem der Ansprüche 1 bis 7, **dadurch gekennzeichnet, dass** das flüchtige Silicon unter den Polydimethylcyclosiloxanen und linearen Polysiloxanen mit niedriger Molmasse ausgewählt ist.

9. Zusammensetzung nach einem der Ansprüche 1 bis 8, **dadurch gekennzeichnet, dass** das flüchtige Silicon ein lineares Polysiloxan vom Hexamethyldisiloxantyp ist.

10. Zusammensetzung nach einem der Ansprüche 1 bis 9, **dadurch gekennzeichnet, dass** sie 55 bis 65 Gew.-% Hexamethyldisiloxan enthält.

11. Zusammensetzung nach einem der Ansprüche 1 bis 10, **dadurch gekennzeichnet, dass** die nichtflüchtige Ölphase ein apolares Öl ist.

12. Zusammensetzung nach einem der Ansprüche 1 bis 11, **dadurch gekennzeichnet, dass** es sich bei dem apolaren Öl um Paraffinöl handelt.

13. Zusammensetzung nach einem der Ansprüche 1 bis 9, **dadurch gekennzeichnet, dass** sie 5 bis 15 % Paraffinöl enthält.

14. Zusammensetzung nach einem der vorhergehenden Ansprüche,
**dadurch gekennzeichnet, dass** sie auch einen Silicongummi enthält.

15. Zusammensetzung nach einem der Ansprüche 1 bis 10, **dadurch gekennzeichnet, dass** sie enthält:
a) 0,3 % (4E,6E)-7-[3-(3,4-Bis-hydroxymethylbenzyloxy)phenyl]-3-ethylnona-4,6-dien-3-ol,
b) 60 % Hexymethyldisiloxan,
c) 10 % Paraffinöl,
d) 29,7 % Ethanol.

16. Zusammensetzung nach einem der Ansprüche 1 bis 11, **dadurch gekennzeichnet, dass** sie enthält:
a) 0,3 % (4E,6E)-7-[3-(3,4-Bis-hydroxymethylbenzyloxy)phenyl]-3-ethylnona-4,6-dien-3-ol,
b) 59,4 % Hexamethyldisiloxan,
c) 0,6 % Silicongummi,
d) 10 % Paraffinöl,
e) 29,7 % Ethanol.

17. Verwendung einer Zusammensetzung nach einem der Ansprüche 1 bis 14 für die Herstellung eines Arzneimittels zur Behandlung von:
- dermatologischen Erkrankungen, die mit einer Keratinisierungsstörung einhergehen, die auf der Differenzierung und Proliferation beruht, insbesondere Acne vulgaris, Acne comedonica, polymorpher Akne, Acne rosacea, nodulocystischer Akne, Acne conglobata, Acne senilis und sekundären Akneformen, wie Acne solaris, Acne medicamentosa oder Acne professionalis;
- Ichthyosis, ichthyosiformen Zuständen, der Darier-Krankheit, Palmoplantarkeratosen, Leukoplasien und leukoplasiformen Zuständen, Lichen der Haut oder der Schleimhaut (oral),
- dermatologischen Erkrankungen mit einer entzündlichen immunoallergischen Komponente, mit oder ohne Störungen der Zellproliferation, insbesondere Psoriasis der Haut, Psoriasis der Schleimhaut oder Psoriasis des Nagels, Psoriasis-Arthritis, Atopie der Haut, wie Ekzemen, respiratorischer Atopie oder gingivaler Hypertrophie,
- benignen oder malignen, dermalen oder epidermalen Proliferationen viraler oder nichtviraler Herkunft, insbesondere Verruca vulgaris, Verruca plana, Epidermodysplasia verruciformis, oraler oder florider Papillomatosis und des T-Lymphoms,
- Proliferationen, die durch UV-Strahlung hervorgerufen sein können, insbesondere Epithelioma basocellulare und spinocellulare,
- präkanzerösen Hautläsionen, insbesondere Keratoacanthoma,
- Immundermatosen, besonders Lupus erythematodes,
- bullösen Immunerkrankungen,
- Kollagenerkrankungen, besonders Sklerodermie,
- dermatologischen oder systemischen Erkrankungen mit immunologischer Komponente,
- Hauterkrankungen, die durch UV-Strahlung verursacht sind, lichtinduzierter oder altersbedingter Hautalterung, aktinischen Keratosen und Pigmentierungen, oder beliebigen Erkrankungen, die mit der altersbedingten oder aktinischen Alterung einhergehen, besonders Xerosis,
- Funktionsstörungen der Talgdrüse, besonders Hyperseborrhoe bei Akne oder Seborrhoe simplex oder seborrhoeische Dermatitis,
- Wundheilungsstörungen oder Dehnungsstreifen,
- Pigmentierungsstörungen, wie Hyperpigmentierung, Melasma, Hypopigmentierung oder Vitiligo,
- Störungen des Lipidstoffwechsels, wie Adipositas, Hyperlipidämie, nicht-insulinpflichtigem Diabetes oder X-Syndrom,
- entzündlichen Erkrankungen wie Arthritis,
- kanzerösen oder präkanzerösen Zuständen,
- Alopezie unterschiedlichen Ursprungs, wie insbesondere durch Chemotherapie oder Strahlung verursachter Alopezie,
- Erkrankungen des Immunsystems, wie Asthma, Diabetes vom Typ 1, multiple Sklerose, oder anderen selektiven Funktionsstörungen des Immunsystems, oder
- Erkrankungen des kardiovaskulären Systems, wie Arteriosklerose oder Bluthochdruck.

18. Verwendung einer Zusammensetzung nach Anspruch 17 für die Behandlung von Psoriasis.

19. Verfahren zur Verbesserung der Penetration eines pharmazeutischen Wirkstoffes, der von Vitamin D abgeleitet ist, **dadurch gekennzeichnet, dass** eine Zusammensetzung mit den folgenden Komponenten in einem pharmazeutisch akzeptablen Träger:
a) eine pharmazeutisch wirksame Menge einer von Vitamin D abgeleiteten Verbindung,
b) mindestens ein flüchtiges Silicon,
c) eine nichtflüchtige Ölphase
auf die Haut aufgebracht wird, wobei die Zusammensetzung als Spray angewandt wird.

20. Verfahren nach Anspruch 19, **dadurch gekennzeichnet, dass** der Wirkstoff das (4E,6E)-7-[3-(3,4-Bis-hydroxymethylbenzyloxy)-phenyl]-3-ethylnona-4,6-dien-3-ol ist, das flüchtige Silicon das Hexamethyldisiloxan ist und das hydrophobe Lösungsmittel Paraffinöl ist.

21. Verfahren nach einem der Ansprüche 19 oder 20, **dadurch gekennzeichnet, dass** die Zusammensetzung ferner einen Silicongummi enthält.

## Revendications

1. Composition comprenant, dans un véhicule pharmaceutiquement acceptable :
a) une quantité thérapeutiquement efficace d'au moins un agent actif pharmaceutique,
b) au moins une silicone volatile,
c) une phase huileuse non volatile,
**caractérisée en ce que** la composition est pulvérisable, et **en ce que** l'agent actif pharmaceutique est dérivé de la vitamine D et est choisi dans le groupe constitué des :
6-[3-(3,4-bishydroxyméthylbenzyloxy)phényl-2-méthylhepta-3,5-dién-2-ol,
7-[3-(3,4-bishydroxyméthylphénoxyméthyl)phényl-3-éthyloctan-3-ol,
7-{3-[2-(3,4-bishydroxyméthylphényl)éthyl]phényl}-3-éthylocta-4,6-dién-3-ol,
6-{3-[2-(3,4-bishydroxyméthylphényl)éthyl]phényl}-2-méthylhepta-3,5-dién-2-ol,
7-{3-[2-(3,4-bishydroxyméthylphényl)vinyl]phényl}-3-éthylocta-4,6-dién-3-ol,
7-[3-(3,4-bishydroxyméthylbenzyloxy)phényl]-3-éthyl-3-octanol,
(4E,6E)-7-[3-(3,4-bishydroxyméthylbenzyloxy)phényl]-3-éthylocta-4,6-dién-3-ol,
(4E,6E)-7-[3-(3,4-bishydroxyméthylbenzyloxy)phényl]-3-éthylnona-4,6-dién-3-ol,
(E)-7-[3-(3,4-bishydroxyméthylbenzyloxy)phényl]-3-éthyloct-4-én-3-ol,
(E)-7-[3-(3,4-bishydroxyméthylbenzyloxy)phényl]-3-éthyloct-6-én-3-ol,
(E)-7-[3-(3,4-bishydroxyméthylbenzyloxy)phényl]-3-éthyloct-6-én-4-yn-3-ol,
(4E,6E)-7-[3-(3,4-bishydroxyméthylphénoxyméthyl)phényl]-3-éthylocta-4,6-dién-3-ol,
(E)-7-[3-(3,4-bishydroxyméthylphénoxyméthyl)phényl]-3-éthylnon-6-én-3-ol,
(E)-7-{3-[3,4-bishydroxyméthylbenzyl)méthylamino]-phényl}-3-éthyloct-6-én-3-ol,
7-[3-(3,4-bishydroxyméthylbenzyloxy)phényl]-3-éthyl-7-méthyloctan-3-ol.

2. Composition selon la revendication 1, **caractérisée en ce qu'**elle comprend :
a) entre 0,0001 et 20 % en poids de l'agent actif,
b) entre 25 et 95 % en poids de silicone volatile,
c) entre 1 et 50 % en poids de phase huileuse non volatile.

3. Composition selon une des revendications 1 ou 2, **caractérisée en ce que** l'agent actif pharmaceutique est le
(4E,6E)-7-[3-(3,4-bishydroxyméthylbenzyloxy)phényl]-3-éthylnona-4,6-dién-3-ol.

4. Composition selon une des revendications 1 à 3, **caractérisée en ce qu'**elle contient entre 0,01 % et 2 % en poids de composé dérivé de vitamine D.

5. Composition selon une des revendications 1 à 4, **caractérisée en ce que** le véhicule est un véhicule alcoolique.

6. Composition selon une des revendications 1 à 5, **caractérisée en ce que** le véhicule alcoolique comprend au moins 15 % d'alcool.

7. Composition selon une des revendications 1 à 6, **caractérisée en ce que** le véhicule alcoolique comprend au moins 25 % d'éthanol.

8. Composition selon une des revendications 1 à 7, **caractérisée en ce que** la silicone volatile est choisie dans le groupe constitué de polydiméthylcyclosiloxanes et de polysiloxanes linéaires à faible poids moléculaire.

9. Composition selon une des revendications 1 à 8, **caractérisée en ce que** la silicone volatile est un polysiloxane linéaire de type hexaméthyldisiloxane.

10. Composition selon une des revendications 1 à 9, **caractérisée en ce qu'**elle contient entre 55 et 65 % en poids d'hexaméthyldisiloxane.

11. Composition selon une des revendications 1 à 10, **caractérisée en ce que** la phase huileuse non volatile est une huile non polaire.

12. Composition selon une des revendications 1 à 8, **caractérisée en ce que** l'huile non polaire est une huile de paraffine.

13. Composition selon une des revendications 1 à 9, **caractérisée en ce qu'**elle contient entre 5 et 15 % d'huile de paraffine.

14. Composition selon l'une quelconque des revendications précédentes, **caractérisée en ce qu'**elle contient en outre une gomme de silicone.

15. Composition selon l'une quelconque des revendications 1 à 10, **caractérisée en ce qu'**elle contient:
a) 0,3 % de (4E,6E)-7-[3-(3,4-bishydroxyméthylbenzyloxy)phényl]-3-éthylnona-4,6-dién-3-ol,
b) 60 % d'hexaméthyldisiloxane,
c) 10 % d'huile de paraffine,
d) 29,7 % d'éthanol.

16. Composition selon l'une quelconque des revendications 1 à 11, **caractérisée en ce qu'**elle contient:
a) 0,3 % de (4E, 6E)-7-[3-(3,4-bishydroxyméthylbenzyloxy)phényl]-3-éthylnona-4,6-dién-3-ol,
b) 59,4 % d'hexaméthyldisiloxane,
c) 0,6% de gomme de silicone,
d) 10 % d'huile de paraffine,
e) 29,7 % d'éthanol.

17. Utilisation d'une composition selon l'une quelconque des revendications 1 à 14, pour produire un produit médicinal destiné à traiter :
- les pathologies dermatologiques associées à un trouble de la kératinisation associé à la différentiation et à la prolifération, en particulier l'acné commune, l'acné comédonienne, l'acné polymorphe, l'acné rosacée, l'acné nodulo-kystique, l'acné conglobata, l'acné sénile, et l'acné secondaire telle que l'acné solaire, médicamenteuse ou professionnelle,
- les ichtyoses, les pathologies ichtyosiformes, la maladie de Darrier, la kératodermie palmo-plantaire, la leucoplasie et les pathologies leucoplasiformes, et le lichen cutané ou muqueux (oral),
- les pathologies dermatologiques avec une composante inflammatoire immuno-allergique, avec ou sans un trouble de prolifération cellulaire, en particulier le psoriasis cutané, muqueux ou unguéal, le rhumatisme psoriasique, une atopie cutanée, telle que l'eczéma, l'atopie respiratoire ou l'hypertrophie gingivale,
- les proliférations dermiques ou épidermiques bénignes ou malignes, d'origine virale ou non virale, en particulier les verrues communes, les verrues plates, l'épidermodysplasie verruciforme, les papillomatoses orales ou florides, et le lymphome T,
- les proliférations qui peuvent être induites par la lumière ultraviolette, en particulier l'épithélioma basocellulaire et l'épithélioma spinocellulaire,
- les lésions cutanées précancéreuses, en particulier les kératoacanthomes,
- les dermatoses immunitaires, en particulier le lupus érythémateux,
- les maladies immunitaires bulleuses,
- les maladies du collagène, en particulier la sclérodermie,
- les pathologies dermatologiques ou systémiques avec une composante immunologique,
- les troubles de la peau dus à une exposition au rayonnement UV, ou au vieillissement solaire ou chronologique de la peau, ou les kératoses et pigmentations actiniques, ou des pathologies quelconques associées au vieillissement chronologique ou actinique, en particulier le xérosis,
- les troubles de la fonction sébacée, en particulier l'acné hyperséborrhéique ou la séborrhée simple ou la dermite séborrhéique,
- les troubles de la cicatrisation ou les marques d'étirement,
- les troubles de la pigmentation, tels que l'hyperpigmentation, le mélasme, l'hypopigmentation ou le vitiligo,
- les pathologies du métabolisme des lipides, telles que l'obésité, l'hyperlipidémie, le diabète de type 2 ou le syndrome X,
- les pathologies inflammatoires telles que l'arthrite,
- les états cancéreux ou précancéreux,
- les alopécies de diverses origines, en particulier l'alopécie causée par une chimiothérapie ou un rayonnement,
- les troubles du système immunitaire, tels que l'asthme, le diabète sucré de type 1, la sclérose en plaques ou d'autres dysfonctionnements sélectifs du système immunitaire, ou
- les pathologies du système cardiovasculaire telles que l'artériosclérose ou l'hypertension.

18. Utilisation d'une composition, selon la revendication 17, pour traiter le psoriasis.

19. Procédé pour améliorer la pénétration d'un agent actif pharmaceutique dérivé de la vitamine D, **caractérisé en ce qu'**une composition comprenant les composants suivants, dans un véhicule pharmaceutiquement acceptable, est appliquée sur la peau :
a) une quantité thérapeutiquement efficace d'un composé dérivé de la vitamine D,
b) au moins une silicone volatile,
c) une phase huileuse non volatile,
ladite composition étant appliquée sous la forme d'un aérosol.

20. Procédé selon la revendication 19, **caractérisé en ce que** l'agent actif est le (4E,6E)-7-[3-(3,4-bishydroxyméthylbenzyloxy)phényl]-3-éthylnona-4,6-dién-3-ol, la silicone volatile est l'hexaméthyldisiloxane, et le solvant hydrophobe est une huile de paraffine.

21. Procédé selon une des revendications 19 et 20, **caractérisé en ce que** la composition comprend en outre une gomme de silicone.
